# EUROPEAN PATENT APPLICATION

(11) **EP 3 777 822 A1**
(43) Date of publication of application: **17.02.2021**
(21) Application number: 19191831.7
(22) Date of filing: 14.08.2019
(51) Int. Cl.: A61K 8/40, A61K 8/49, A61Q 5/00, A61Q 5/06, A61Q 5/10, A61K 8/41

(54) **COSMETIC COMPOSITION**

(71) Applicant: HCT - Hair Cosmetic Technology AG, 1723 Marly (CH)
(72) Inventor: GÖTTEL, Otto Richard, 1723 Marly (CH); AEBY, Johann, 1723 Marly (CH)
(74) Representative: Herzog IP Patentanwalts GmbH

(57) **Abstract**

The present invention relates to a cosmetic composition comprising at least these components i. a first and a second primary intermediate, and ii. a first and a second coupling agent; wherein the first primary intermediate is 2,4,5,6-tetraaminopyrimidine or a salt thereof; wherein the second primary intermediate is selected from the group consisting of p-aminophenol, 4-amino-m-cresol and salts thereof, or a combination of two or more of these; wherein the first coupling agent is 5-amino-6-chlor-o-cresol; and wherein the second coupling agent is selected from the group consisting of 2-amino-3-hydroxypyridine, 6-amino-m-cresol, 2-amino-5-ethylphenol and salts thereof, or a combination of two or more of these; the present invention further refers to a kit of two or more individually packaged components for coloring keratin fibers, to a ready-to-use composition obtainable by mixing the kit components, to a process for coloring keratin fibers comprising said ready-to-use composition and to a use of the cosmetic composition.

## Description

The present invention relates to a cosmetic composition suited for dyeing keratin fibers, in particular human hair, comprising at least these components i. a first and a second primary intermediate, and ii. a first and a second coupling agent; wherein the first primary intermediate is 2,4,5,6-tetraaminopyrimidine or a salt thereof; wherein the second primary intermediate is selected from the group consisting of p-aminophenol, 4-amino-m-cresol and salts thereof, or a combination of two or more of these; wherein the first coupling agent is 5-amino-6-chloro-o-cresol; and wherein the second coupling agent is selected from the group consisting of 2-amino-3-hydroxypyridine, 6-amino-m-cresol, 2-amino-5-ethylphenol and salts thereof, or a combination of two or more of these; the present invention further refers to a kit of two or more individually packaged components for coloring keratin fibers, to a ready-to-use composition obtainable by mixing the kit components, to a process for coloring keratin fibers comprising said ready-to-use composition and to a use of the cosmetic composition.

It is known to dye keratin fibers, and in particular human hair, with dye compositions containing oxidation dye precursors, the so-called primary intermediates (also referred to as: oxidation bases) and the so-called coupling agents. The primary intermediates employed in these cosmetic compositions are usually derivatives of benzene, e.g. p-phenylenediamines, o- and p-amino phenols or heterocyclic compounds such as 4,5-diamino pyrazoles. The coupling agents are usually selected from aromatic meta-diamines, meta-aminophenols, meta-diphenols (resorcinols), certain heterocyclic compounds and derivatives thereof.

The oxidation dye precursors are usually colorless or weakly colored compounds which, when combined with oxidizing products such as hydrogen peroxide compositions, can be converted to colored compounds and dyes by a process of oxidative condensation. It is also known that the shades obtained with these primary intermediates can be varied by combining them with color couplers. The variety of compounds used as regards the oxidation bases and the couplers allows a wide range of colors to be obtained.

Within the broad color palette, natural shades are by far the most important shades. Commercial products providing natural shades based on oxidation dyes, comprising at least a p-phenylenediamine dye intermediate having unsubstituted amino groups, for instance p-phenylenediamine, toluene-2,5-diamine, 2-hydroxyethyl-p-phenylenediamine and 2-methoxy-methyl-p-phenylenediamine as primary intermediate and at least a meta-diphenol as coupler.

In the field of oxidation dyeing of hair, primary intermediates and coupling agents are in general sought which allow a coloration having satisfactory resistance to light, to washing, to bad weather, to perspiration and to the various treatments to which hair may be subjected to be imparted to the hair.

However, numerous oxidation dye intermediates, mainly p-phenylenediamines, have been criticized because of their skin sensitization potential. As a consequence, some of these substances have to be declared on the packaging of the products. Thus, the packaging of hair colors, have to indicate a warning hint which expressly points out the presence of the dye precursors resorcinol, diaminotoluene and diaminobenzene. Another substance with similar declaration requirements is ammonia which is often found as well as a component in dyeing formulations.

The applicant aims to provide hair coloring and hair shading compositions which are friendlier to the customers skin and hair, and also less disputed. In particular, the applicant sought to provide cosmetic compositions, e.g. for dyeing keratin fibers, which are free from substances that are required to be declared by warning marks on the product containers. More specifically, the applicant searched a way to provide a cosmetic composition which is free from the widely used p-phenylenediamine and toluenediamine. Moreover, the composition should be also free from resorcinol. Further, it should be feasible to provide an oxidation dye system which does not require any of the above-mentioned warnings.

In the field of new p-phenylenediamine derivatives, considerable progress has been made with the invention of 2-methoxy-methyl-p-phenylenediamine (EP1103542B1) which combines both, low sensitization potential and performance. However, it is still unsatisfactory because also 2-methoxy-methyl-p-phenylenediamine is broadly used in combination with resorcinol to produce natural shades.

Other specific oxidation dye formulations free from p-phenylenediamines and resorcinols are known, for instance combinations of 4,5-diamino-1-hydroxyethyl pyrazole sulfate or 1-hexyl 4,5-diamino pyrazole sulfate with various couplers. However, both pyrazoles have the disadvantage that they cover the orange to violet-blue range, but not the natural brown or black shades which are mostly desired by the customers.

So far, the hair cosmetic industry was not able to provide oxidation dye combinations for covering the broad palette of stable natural shades which are free from p-phenylenediamines and resorcinols. Accordingly, there is both an ongoing need and search, for further developing cosmetic hair coloring systems for natural looking brown and black shades free from p-phenylenediamines and resorcinols.

While exploring the potential of 2,4,5,6-tetraaminopyrimidine sulfate, the applicant has found that numerous combinations with standard hair dye couplers lead to colorations which have very disappointing properties with regard to storage stable tint compositions, long-term performance and color stability of the obtained shades. So, for instance it was found that combinations with the widely used blue couplers 2,4-diaminophenoxyethanol HCl and 2-amino-4-hydroxyethylaminoanisole sulfate, which are standard in conventional systems with p-phenylenediamines, gave intense blue dyeouts directly after dyeing, but the colors change progressively to green within a 1-2 weeks period. Another species of couplers such as m-aminophenol or the heteroyclic 6-methoxy-2-methylamino-3-aminopyridine exhibited similar results. Due to the observed instability more complex dye combinations comprising these compounds do also exhibit considerable color shifts over a relatively short period and are therefore not satisfactory. Further, the broadly used coupling agents 4-amino-2-hydroxytoluene and 2-methyl-5-hydroxyethylaminophenol, which are normally added for producing red shades, are not entirely stable in the inventive system and can therefore not be used either.

Despite all efforts of the past to provide cosmetic hair coloring compositions which satisfy the customers' as well as the hairdressers' needs, there is an ongoing need for further development in this area to satisfy the markets' needs.

Accordingly, it is an object of the invention to provide a cosmetic composition which overcomes at least one, preferably two or more of the disadvantages which were described with regard to art.

Given the above, it was completely unexpected that the specific the combination of 2,4,5,6-tetraaminopyrimidine with 5-amino-6-chloro-o-cresol, which provides the blue component in a more complex composition, is suited to prepare natural shades and exhibits the required stability leading to colorations with the endurance of conventionally dyed hair.

Another object of the invention is to provide a cosmetic composition for the treatment of hair, in particular coloring, which is well accepted by model home users and clients of hair dressers.

Another object of the invention is to provide a cosmetic composition which is not irritating to skin or mucous membranes of model home users and clients of hair dressers.

Another object of the invention is to provide a cosmetic composition which is stable when in stock for an extended period of time.

Another object of the invention is to provide a cosmetic composition which can be applied to, worked on and rinsed off the keratin fibers, e.g. hair, with ease.

Another object of the invention is to provide a cosmetic composition which does not cause significant damage to keratin fibers.

### EMBODIMENTS OF THE INVENTION

The invention is described in the form of embodiments in this section.
|1| A cosmetic composition comprising at least these components
   i. a first and a second primary intermediate, and
   ii. a first and a second coupling agent;
      wherein the first primary intermediate is 2,4,5,6-tetraaminopyrimidine or a salt thereof;
      wherein the second primary intermediate is selected from the group consisting of p-aminophenol, 4-amino-m-cresol and salts thereof, or a combination of two or more of these group members;
      wherein the first coupling agent is 5-amino-6-chlor-o-cresol; and
      wherein the second coupling agent is selected from the group consisting of 2-amino-3-hydroxypyridine, 6-amino-m-cresol, 2-amino-5-ethylphenol and salts thereof, or a combination of two or more of these group members.
|2| The cosmetic composition according to embodiment 111, wherein the composition comprises at least one of these features:
   a) less than 0.1wt.% of a phenylene diamine or salts thereof;
   b) less than 0.1wt.% of a resorcinol; or
   c) less than 0.2wt.% both, a) and b).
|3| The cosmetic composition according to any one of the preceding embodiments, comprising at least a further coupling agent selected from the group consisting of these group members: 3-amino-2,6-dimethylphenol, hydroxyethyl-3,4-methylenedioxyaniline and salts thereof, or a combination of two or more of these group members.
|4| The cosmetic composition according to any one of the preceding embodiments comprising at least one direct dye.
|5| The cosmetic composition according to embodiment 4, wherein the at least one direct dye is selected from the group consisting of these group members: 2-amino-6-chloro-4-nitrophenol, 4-nitro-o-phenylenediamine, 2,6-diamino-3-((pyridine-3-yl)azo)pyridine, and salts thereof, or a combination of two or more of these group members.
|6| The cosmetic composition of any one of the preceding embodiments, wherein the composition further comprises at least one organic phosphate ester compound.
|7| The cosmetic composition of embodiment |6|, wherein at least one organic phosphate ester compound is selected from the group consisting of
   c1. monoester of phosphates of alkoxylated fatty alcohols,
   c2. diester of phosphates of non-alkoxylated fatty alcohols, w
      herein the non-alkoxylated fatty alcohols are composed of C₁₂ - C₂₂ fatty alcohols;
   c3. mixtures of c1 and c2.
|8| The cosmetic composition of any one of the preceding embodiments, wherein the composition further comprises at least one alkaline agent.
|9| The cosmetic composition of any of the preceding embodiments, wherein the composition has at least one of these features:
   a. A cumulative amount of all primary intermediates in the range from 0.0001 to 10 wt.%, the wt.% based on the weight of the total composition;
   b. A cumulative amount of all coupling agents in the range from 0.0001 to 10 wt.%, the wt.% based on the weight of the total composition;
   c. A ratio of the cumulative amounts of all primary intermediates and all coupling agents in the range of from 3:1 to 1:3, based on molar amounts;
   d. A cumulative amount of all alkaline agents in the range from 0.1 to 17.5 wt.%, the wt.% based on the weight of the total composition;
   e. A cumulative amount of all organic phosphate ester compounds in the range from 0.1 to 5.0 wt.%, the wt.% based on the weight of the total composition;
      Or a combination of any two or more of the features a. - e.
|10| A kit for coloring keratin fibers, comprising in individually packaged form at least two kit components:
   I) a cosmetic composition as described in any one of embodiments |1| to |9|
   II) a developer composition comprising an oxidizing agent.
|11| The kit according to embodiment |10|, wherein the oxidizing agent comprises an aqueous solution of hydrogen peroxide.
|12| A ready-to-use composition obtainable by mixing the kit components according to any one of embodiments |10| or |11|.
|13| The ready-to-use composition according to embodiment |12|, wherein the ratio of component I and component II is in the range of from 2:1 up to 1:3, each number based on parts by weight.
|14| A process for coloring keratin fibers, comprising the steps of:
   I. providing keratin fibers;
   II. contacting the keratin fibers of step I. with the ready-to-use composition according to any one of embodiments |12| or |13|, and allowing the ready-to-use composition to remain on the keratin fibers for a period of time;
   III. optionally rinsing the keratin fibers;
   IV. optionally drying the keratin fibers.
|15| A use of a cosmetic composition according to anyone of the embodiments |1| to |9| for coloring keratin fibers, such as human or pet hair, which are free of resorcinols and phenylene diamines.

### DESCRIPTION OF THE INVENTION

A contribution to the solution of at least one of the above objects is provided by the subject-matter of the category-forming claims. The dependent sub-claims of the category-forming claims represent preferred embodiments of the invention, the subject-matter of which also makes a contribution to solving at least one of the objects mentioned above.

A first aspect of the invention is a cosmetic composition comprising at least these components
i. a first and a second primary intermediate, and
ii. a first and a second coupling agent;
   wherein the first primary intermediate is 2,4,5,6-tetraaminopyrimidine or a salt thereof;
   wherein the second primary intermediate is selected from the group consisting of these group members: p-aminophenol, 4-amino-m-cresol and salts thereof, or a combination of two or more of these group members;
   wherein the first coupling agent is 5-amino-6-chlor-o-cresol; and wh
   erein the second coupling agent is selected from the group consisting of these group members: 2-amino-3-hydroxypyridine, 6-amino-m-cresol, 2-amino-5-ethylphenol and salts thereof, or a combination of two or more of these group members.

A cosmetic composition in the context of the present invention refers to a composition which can be used for treatment of keratin fibers, such as hair. It can be used for lightening and/or coloring of keratin fibers. The cosmetic composition of the present invention is a liquid, a cream, a gel, a dispersion or a paste, and the like.

A salt of a chemical compound in the present context refers to an anion or cation of this chemical compound. It often has an inorganic counter ion, e.g. Na⁺, K⁺, Mg²⁺, Cl⁻. Br⁻, SO₄²⁻, SO₄Me⁻.

The cosmetic composition can comprise one or more of these features:
a) less than 0.1wt.%, for example less than 0.05 wt.% of a phenylene diamine or salts thereof;
b) less than 0.1wt.%, for example less than 0.05 wt.% of a resorcinol; or
c) less than 0.2wt.%, for example less than 0.1 wt.% of both, a) and b).

Further, the cosmetic composition may comprise no phenylene diamine, phenylene diamine derivative or salt thereof. In other words, the cosmetic composition may be free of any of these compounds. Further, the cosmetic composition may comprise no resorcinol or resorcinol derivative. In other words, the cosmetic composition may be free of resorcinols. Further, the cosmetic composition may comprise no phenylene diamine, no derivative or salt thereof and no resorcinol or derivative thereof. In this event, the cosmetic composition is free of all these compounds.

The cosmetic composition may comprise at least a further coupling agent. The at least one further coupling agent is different from the first and the second coupling agent. The at least one further coupling agent can be selected from the group consisting of these group members: 3-amino-2,6-dimethylphenol, hydroxyethyl-3,4-methylenedioxyaniline, salts thereof, or a combination of two or more of these group members.

In another embodiment, the cosmetic composition may optionally comprise at least one direct dye. The at least one direct dye can be selected by preference from the group consisting of these group members: 2-amino-6-chloro-4-nitrophenol, 4-nitro-o-phenylenediamine, 2,6-diamino-3-((pyridine-3-yl)azo)pyridine. Moreover, the at least one direct dye can be a salt of one of the aforementioned chemical compounds. Further, combinations of two or more of such salts, and combinations of at least one direct dye and a salt of one of the direct dyes can be employed. Moreover, a combination of a direct dye and its salt, e.g. the chloride salt, bromide salt, sulfate salt or methylsulfate salt thereof, can be employed in the cosmetic composition.

The cosmetic composition of any one of the preceding claims, wherein the composition further comprises at least one organic phosphate ester compound. For example, the at least one organic phosphate ester compound selected from
c1. monoester of phosphates of alkoxylated fatty alcohols,
c2. diester of phosphates of non-alkoxylated fatty alcohols,
   wherein the non-alkoxylated fatty alcohols are composed of C₁₂ - C₂₂ fatty alcohols;
c3. mixtures of c1 and c2.

In a preferred embodiment of the invention, the cosmetic composition comprises from 0.1 to 6 wt.%, or from 0.5 to 4 wt.%, or from 1 to 2.5 wt.% in total of the one or more organic phosphate ester compounds, each wt.% based on the total weight of the cosmetic composition.

Turning to the chemical identity of the organic phosphate ester compound, numerous organic phosphate ester compounds with the aforementioned features are known in the art and appear useful in the present invention.

The monoester of phosphates of alkoxylated fatty alcohols of the composition according to the invention are composed of C₁₂ - C₂₂ fatty alcohols alkoxylated with from 1 to 50 moles of an alkylene oxide, the number of moles of alkylene oxide with respect to the moles of fatty alcohol. Formula (1) is a general representation of an organic phosphate ester.

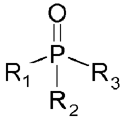

A monoester of phosphate of one or more alkoxylated fatty alcohols according to the invention is characterized as follows:
Rₓ with x = 1,2,3 can be same or different and Rₓ is selected from:
aa) -OM, wherein M equals H, Na or K;
bb) -OR₄, wherein R₄ can be linear or branched and is a C₁ - C₄₀ alkyl group, preferably C₁₂ - C₂₂, or a C₂ - C₄₀ alkenyl group, preferably C₁₂ - C₂₀;
cc) -(OCH₂CH₂)ₙ(OCH₂CH(CH₃))ₘOR₄, wherein R₄ has the same meaning as identified above, n is an integer in the range of from 1 to 50;
with the proviso that at least one group Rₓ is chosen according to aa) and at least another group is chosen according to bb) or cc).

In a further preferred embodiment, the monoester of phosphate is linear, more preferably it is defined by at least one alkyl group R₄, wherein R₄ is selected from the group consisting of C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, R₄ being preferably one of C₁₆, C₁₈, C₂₀, C₂₂.

In a further preferred embodiment, the monoester of phosphate is linear, more preferably it is defined by at least one alkenyl group R₄, wherein R₄ is selected from the group consisting of C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, R₄ being preferably one of C₁₆, C₁₈, C₂₀, C₂₂.

The diester of phosphates of non-alkoxylated fatty alcohols of the composition according to the invention are composed of C₁₂ - C₂₂ non-alkoxylated fatty alcohols. With respect to formula (1), diester of phosphates of non-alkoxylated fatty alcohols according to the invention are characterized as follows:
Rₓ with x = 1,2,3 can be same or different, and Rₓ is selected from:
aa) - OM, wherein M equals H, Na or K;
bb) - OR₄, wherein R₄ can be linear or branched and is a C₁ - C₄₀ alkyl group, preferable C₁₂ - C₂₂, or a C₂ - C₄₀ alkenyl group, preferable C₁₂ - C₂₀;
with the proviso that one group Rₓ is chosen according to aa) and two groups are chosen according to bb).

In a further preferred embodiment, the monoester of phosphate is linear, more preferably it is defined by at least one alkyl group R₄, wherein R₄ is selected from the group consisting of C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, R₄ being preferably one of C₁₆, C₁₈, C₂₀, C₂₂.

In a further preferred embodiment, the monoester of phosphate is linear, more preferably it is defined by at least one alkenyl group R₄, wherein R₄ is selected from the group consisting of C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, R₄ being preferably one of C₁₆, C₁₈, C₂₀, C₂₂.

Moreover, mixtures of the abovementioned phosphate esters can be employed. These mixtures can comprise two or more monoester of phosphates of alkoxylated fatty alcohols and one diester of phosphate of non-alkoxylated fatty alcohols, one monoester of phosphates of alkoxylated fatty alcohols and two or more diesters of phosphate of non-alkoxylated fatty alcohols, or a mixture of two or more monoesters of phosphates of alkoxylated fatty alcohols and two or more diesters of phosphate of non-alkoxylated fatty alcohols.

In a further preferred embodiment of the invention, the at least one organic phosphate ester compound is selected from the group consisting of dicetyl phosphate, ceteth-10 phosphate, oleth-5 phosphate and dioleyl phosphate. Yet more preferred is a combination of two or more of these phosphate esters, or even all of them. All names are provided according to INCI nomenclature.

Some preferred combinations of the above phosphate esters are commercially available from Croda GmbH (41334 Nettetal, Germany) under the trade name CRODAFOS. These are, e.g.
- Ceteth-10 phosphate und Dicetyl phosphate, as in CRODAFOS CES,
- Ceteth-20 phosphate und dicetyl phosphate, as in CRODAFOS CS-20 ACID, and
- Oleth-5 phosphate und Dioleyl phosphate, as in CRODAFOS HCE.

In a particularly preferred embodiment of the invention, the organic phosphate ester compound comprises at least Dicetyl Phosphate and Ceteth-10 Phosphate. A commercial product which is very suitable and fulfils this requirement is Crodafos CES®. Crodafos CES® is available for purchase from Croda International Plc, Snaith (United Kingdom). Crodafos CES® comprises these three components: Cetearyl alcohol (mixture of Cetyl alcohol and Stearyl alcohol), and dicetyl phosphate (CAS: 2197-63-9) and ceteth-10 phosphate (CAS : 50643-20-4).

In a preferred embodiment of the invention, the first kit component comprises from 0.1 to 10 wt,-%, preferably 0.1 to 6 wt.%, or from 0.5 to 4 wt.%, or from 1 to 2.5 wt.% in total of an organic phosphate ester compound comprising at least Dicetyl Phosphate and Ceteth-10 Phosphate, wherein the wt.% are the combined wt-% of all organic phosphate ester compounds in the first kit component, and the wt.-% are based on the total weight of the first kit component.

In a further preferred embodiment of the invention, the first kit component comprises from 0.1 to 10 wt,-%, preferably 0.1 to 6 wt.%, or from 0.5 to 4 wt.%, or from 1 to 2.5 wt.% in total of Crodafos CES ®, the wt.-% are based on the total weight of the first kit component.

The above embodiment is in no way limiting and only serves as an example of useful commercial products in general. Other carriers are known and can be used in a similar manner. The alternative carriers as well as typical formulations are known to a person skilled in the art.

The above mentioned exemplary cosmetic compositions as well as all further cosmetic compositions within the above framed scope can be used. In particular compositions manufactured therewith can comprise fatty alcohols and/or alkoxylated fatty alcohols, each with from 8 to 30 carbon atoms. Examples of these further fatty alcohols and/or alkoxylated fatty alcohols are Cetyl alcohol, Stearyl alcohol and mixtures thereof, Octyldodecanol, 2-Butyloctanol, 2-Hexyldecanol, 2-Undecylpentadecanol, Oleyl alcohol and Linoleyl alcohol (all according to INCI nomenclature).

In another embodiment, the cosmetic composition may comprise fatty acids, e.g., oleic acid or a salt thereof. These compositions may further comprise one or more aliphatic alcohols, such as ethanol and/or isopropanol.

A further component of the cosmetic composition according to the invention is water, preferably, demineralized water.

The cosmetic composition may further comprise at least one alkaline agent. All alkaline agents can be employed which are known in the art and appear suitable for the preparation of the composition of the invention. In a further preferred embodiment, the alkaline agent is selected from the group consisting of ammonium hydroxide, an alkanolamine such as monoethanolamine (MEA), diethanolamine, triethanolamine, monopropanolamine, dipropanolamine, tripropanolamine, 2-amino-2-methyl-1,3-propanediol (AMPD), 2-amino-2-ethyl-1,3-propanediol (AEPD), 2-amino-2-methyl-1-propanol (AMP), 2-amino-2-hydroxymethyl-1,3-propanediol (TRIS), sodium and potassium hydroxide, ammonium carbonate, ammonium bicarbonate, ammonium carbamate, and a combination of two or more thereof.

The cosmetic composition can further comprise at least one amino acid. In general, all amino acids can be employed which are known in the art. In the present context, the term amino acid includes free amino acids, salts of amino acid, e.g. sodium or potassium salt with regard to the cation, as well as halides on behalf of the anion, if applicable. Preferred amino acids are selected from the group consisting of glycine, serine, asparagine, threonine, glutamine, arginine and lysine as well as α- and β-alanine, and mixtures of two or more compounds thereof. Smaller amino acids are more preferred than larger ones; wherein small and large refer to the molecular weight of the amino acid.

In a preferred embodiment of the invention, the cosmetic composition comprises a total of from 1.0 to 30 wt.%, preferably from 1.0 to 20 wt.%, or from 3.0 to 15 wt.%, or from 0.5 to 10 wt.%, or from 6 to 12 wt.% of one or more amino acids, each wt.% based on the total weight of the cosmetic composition.

The cosmetic composition may further have at least one of these features:
a. A cumulative amount of all primary intermediates in the range from 0.0001 to 10 wt.%, for example from 0.001 to 8 the wt.%, or from 0.001 to 5 wt.%, based on the weight of the total composition;
b. A cumulative amount of all coupling agents in the range from 0.0001 to 10 wt.%, for example from 0.001 to 8 the wt.%, or from 0.001 to 5 wt.%, the wt.% based on the weight of the total composition;
c. A ratio of the cumulative amounts of all primary intermediates and all coupling agents in the range of from 3:1 to 1:3, for example in the range from 2:1 to 1:2, or about 1:1, always based on the molar ration of the compounds [mol/mol].
d. A cumulative amount of all alkaline agents in the range from 0.1 to 17.5 wt.%, or from 2 to 10 wt.-%, yet more preferable from 2 to 8 wt.-%., the wt.% based on the weight of the total composition;
e. A cumulative amount of all organic phosphate ester compounds in the range from 0.1 to 5.0 wt.%, or from 0.5 to 4 wt.%, or from 1 to 2.5 wt.%, the wt.% based on the weight of the total composition.

Combinations of two or more of the features from a.-e. may be preferred. For example, a combination of the features a.b., a.c., a.d., a.e., b.c., b.d., b.e., c.d, c.e., d.e. may represent preferred combinations. Further the features combinations a.b.c., a.b.d., a.b.e., a.c.e., a.d.e., b.c.d., b.c.e., b.d.e., c.d.e., a.b.c.d., a.b.c.e., a.c.d.e., a.b.d.e., b.c.d.e. and a.b.c.d.e. are preferred.

The cosmetic composition of the invention can comprise further components. Amongst these, direct polymers, natural or vegetable oils, organic solvents, complex forming agents, acids and bases to regulate pH and antioxidants are most common.

A second aspect of the invention is a kit for coloring keratin fibers, comprising in individually packaged form at least two kit components:
I a cosmetic composition as claimed in the first aspect of the invention and embodiments thereof; and
II a developer composition comprising an oxidizing agent.

In a preferred embodiment, the oxidizing agent of the kit comprises an aqueous solution of hydrogen peroxide. In a further preferred embodiment, the amount of hydrogen peroxide in the aqueous solution is in the range of from 2 to 12 wt.%, with respect to the total weight of the aqueous solution.

Oxidative hair coloring compositions like those according to the present invention are usually sold in kits comprising, in individually packaged components such as separate containers, a first container containing the tint component comprising dyes, and an alkaline agent and; the second container containing a developer composition comprising the oxidizing agent (usually hydrogen peroxide). The consumer mixes the content of the first container and the second container together immediately before use, thereby, obtaining a ready-to-use composition and applies it onto the hair.

A third aspect of the invention is a ready-to-use composition obtainable by mixing the kit components described in the second aspect of the invention. Preferably, the kit components are mixed directly prior to application to the keratin fibers, e.g. hair. This enables best performance of the ready-to-use composition.

In a preferred embodiment, the ratio of the kit components I and II is in the range of from 3:1 and 1:3, for example from 1:2 to 2:1, the amounts in the ratios based on parts by weight of components I and II.

After working the ready-to-use composition for a few minutes (to ensure uniform application to all of the hair), a ready-to-use composition is allowed to remain on the hair for an amount of time sufficient for obtaining the target shade. The remaining period is in the range of from 5 to 90 minutes, preferably 10 to 60 minutes, and usually about 30 minutes.

In the kit composition of the invention, a third container may be present. In this event, all three components can either be mixed immediately before use and applied together. Preferably this procedure is carried out if, for instance, the third container comprises a dye which does not support conditions present in the tint (e.g. reducing conditions). Alternatively, the content of the third container is applied after an optional rinse step immediately after processing as a post-treatment; in such cases the container comprises a conditioner.

A fourth aspect of the invention is a process for coloring keratin fibers, comprising the steps of:
I. providing keratin fibers;
II. contacting the keratin fibers of step I. with the ready-to-use composition described in the third aspect of the invention and allowing the ready-to-use composition to remain on the keratin fibers for a period of time, sufficient to obtain the desired color result;
III. optionally rinsing the keratin fibers;
IV. optionally drying the keratin fibers.

Numerous kinds of keratin fibers are known to those skilled in the art. Preferred keratin fibers in the context of the present invention are human hair and animal hair. The process for coloring keratin fibers is described in the following with regard to human hair. This is not intended to limit the scope of the claimed process. To the contrary, it is understood that the process can be applied in the same way to any other kind of keratinous material.

Oxidative hair coloring compositions like those according to the present invention are usually sold in kits comprising, in individually packaged components such as separate containers, a first container containing the dye component comprising the oxidative dye, precursors and a base and; the second container containing a developer composition comprising the oxidizing agent (usually hydrogen peroxide). The consumer mixes the content of the first container and the second container together immediately before use thereby obtaining a ready-to-use composition and applies it onto the hair.

After processing the ready-to-use composition (to ensure uniform application to all of the hair), the ready-to-use composition is allowed to remain on the hair for an amount of time sufficient for the dyeing to take place. The remaining period is in the range of from 5 to 90 minutes, preferably 10 to 60 minutes, and usually about 30 minutes. The consumer then rinses his/her hair thoroughly with tap water and allows it to dry. It is observed that the hair has changed from its original color to the desired color.

When present in the composition of the invention, the optional hair care agent can be provided in an additional, e.g, a third container. In the latter case, all three compositions can be mixed immediately before use and applied together, or the content of the third container can be applied (after an optional rinse step) as a post-treatment immediately after the ready-to-use composition resulting from the mixture of the other containers.

A fifth aspect of the invention is a use of a cosmetic composition according to anyone of the embodiments according to the first aspect for coloring keratin fibers, such as human or pet hair, which are free of resorcinols and phenylene diamines.

### FIGURES

- Fig. 1: shows a kit for coloring keratin fibers consisting of 1 tube and 1 applicator bottle.
- Fig. 2: exhibits a process for preparing a ready-to-use composition.
- Fig. 3: shows a process for coloring keratin fibers.
- Fig. 4: is a schematic of model head.

### DESCRIPTION OF THE FIGURES

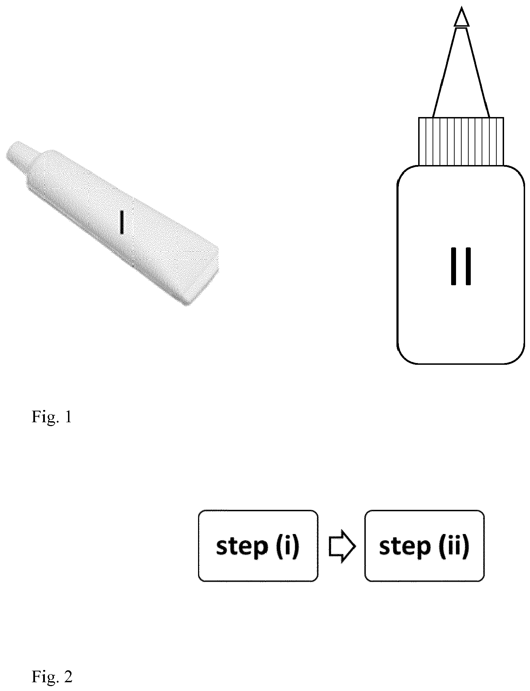

Figure 1 shows a kit for coloring keratin fibers consisting of 2 tubes with cream wherein tube I comprises a first kit component and tube II comprises a second kit component. The second kit component can be a composition as manufactured according to Figure 1.

Fig. 2 exhibits a process for preparing a ready-to-use composition. The process comprises at least two steps. In step (i), a kit as in Figure 1 is provided. The two tubes I and II are opened and the first and second kit component is dispensed into a vessel, e.g. a bowl or an applicator bottle. Then, the first and second kit components are mixed in step (ii) which results in a ready-to-use composition.

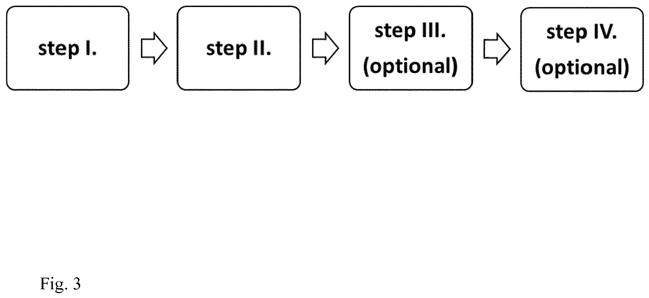

Figure 3 shows a process for coloring keratin fibers, comprising these steps: I. providing keratin fibers; II. contacting the keratin fibers of step I. with a ready-to-use composition which was obtained by mixing the components of a kit as in figure 1., further allowing the ready-to-use composition to remain on the keratin fibers for a period of time; III. (optionally) rinsing the keratin fibers; IV. (optionally) drying the keratin fibers.

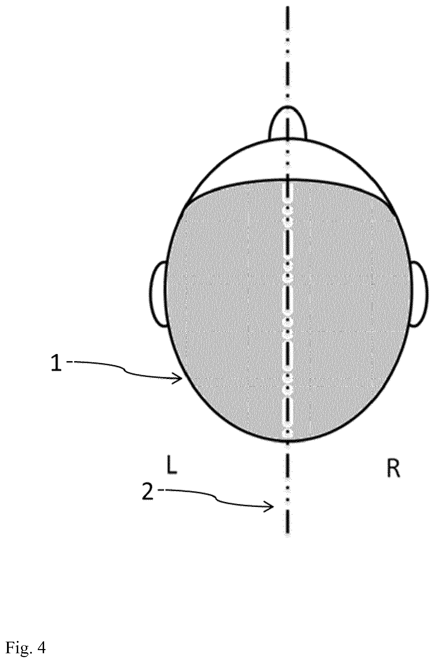

Figure 4 is a schematic of model head 1. The left side L of the head with hair (not shown here) was treated with a reference coloring composition, the right side R of the head with hair (not shown here) was treated with a composition obtained from mixing the two component kit under evaluation.

### EXAMPLES

The following examples illustrate some aspects of the invention. It is understood that the examples and embodiments described herein are for illustrative purposes only and that various modifications or changes in light thereof may be suggested by one skilled in the art without departing from the scope of the present invention.

Amounts mentioned in the tables below refer to wt.-% if not indicated to the contrary. When referring to rinsing or washing of hair with water in the examples, this is tap water, with a hardness of dH = 8.4 (equals 1.5 mmol CaCO₃/liter H₂O). When referring to water as component of a composition (in tables, denoted as "aqua"), this is demineralized water as used for cosmetic purposes.

### Examples 1-3

Examples 1, 2 and 3 have been prepared based on a standard formulation. These examples are not reflective of the invention.

**Table 1: developer compositions with hydrogen peroxide as oxidation agent**

| Developer composition | 1 | 2 | 3 |
|---|---|---|---|
| Cetearyl alcohol | 2.0 | 2.0 | 3.50 |
| Ceteareth-50 | 0.5 | 0.5 | 1.0 |
| Phosphoric acid 85% | 0.1 | 0.1 | 0.1 |
| Hydrogen peroxide 35% | 17.1 | 25.8 | 17.1 |
| Polysorbate-20 | - | - | 2.00 |
| Aqua ad | 100.0 | 100.0 | 100.0 |
| Hydrogen peroxide in the developer composition | 6% | 9% | 6% |
| pH of the developer composition | 3.0 | 3.0 | 3.0 |

**Table 2: Tint compositions (cream)**

| Tint composition | 1 | 2 | 3 | 4 | 5 |
|---|---|---|---|---|---|
| Crodaphos CES* | 6.00 | 8.00 | 7.00 | 6.50 | 7.50 |
| Cetearyl alcohol | 4.00 | - | - | 3.50 | - |
| Lauryl alcohol | - | - | 1.00 | - | 1.00 |
| Stearyl alcohol | - | 3.00 | - | - | - |
| Ceteareth-25 | 0.20 | - | - | 0.20 | - |
| Ceteareth-50 | - | - | 0.40 | - | 0.40 |
| Steareth-21 | - | 0.50 | - | - | - |
| Xanthan gum | 0.10 | 0.40 | 0.20 | 0.10 | 0.20 |
| Propylene glycol | 3.00 | 5.00 | 3.00 | 3.00 | 3.00 |
| Glycine | - | 8.00 | - | - | - |
| Arginine | - | 1.00 | - | - | - |
| Sodium hydroxide | - | 2.42 | - | - | - |
| Ascorbic acid | 0.30 | 0.30 | 0.30 | 0.30 | 0.30 |
| Sodium sulfite | 0.30 | 0.30 | 0.30 | 0.30 | 0.30 |
| EDTA | 0.20 | 0.20 | 0.20 | 0.20 | 0.20 |
| 2,4,5,6-Tetraaminopyrimidine sulfate | 3.50 | 0.80 | 0.40 | 3.50 | 0.02 |
| 5-Amino-6-chloro-o-cresol | 2.00 | 0.40 | 0.20 | 2.40 | 2.00 |
| 2-Amino-3-hydroxypyridine | 0.45 | 0.90 | 0.21 | 0.11 | 0.41 |
| 4-Amino-m-cresol | 0.30 | 0.95 | 0.20 | - | 2.00 |
| 2-Amino-5-ethylphenol HCl | - | 0.50 | - | - | - |
| 2-Amino-6-chloro-4-nitrophenol | 0.30 | - | 0.025 | | |
| Ammonia, 28% in water | 4.50 | - | - | 3.0 | 8.00 |
| Monoethano lamine | - | - | 4.00 | - | - |
| | | | | | |
| Aqua, ad | 100 | 100 | 100 | 100 | 100 |

| | | | | | |
|---|---|---|---|---|---|
| *Crodafos CES is available from Croda International Plc, Snaith, United Kingdom. | | | | | |

Tint composition 1 (according to table 2) was mixed in 1:1 ratio with developer composition 1 (according table 1) and applied onto medium-blond human hair. After remaining on the hair for 30 minutes, the hair was rinsed with water (T_{water} = 30°C) and dried. The color of the treated hair was black.

Tint composition 2 (according to table 2) was mixed in 1:1 ratio with developer composition 2 (according table 1) and applied onto human hair with 50 % grey hair. After remaining on the hair for 30 minutes, the hair was rinsed with water (T_{water} = 30°C) and dried. The color of the treated hair was dark-blond.

By means of an applicator bottle, tint composition 3 (according to table 2) was mixed in 1:1.5 ratio with developer composition 3 (according table 1) and applied onto human hair with 50 % grey hair. After remaining on the hair for 30 minutes, the hair was rinsed with water (T_{water} = 30°C) and dried. The color of the treated hair was light-blond.

Tint composition 4 (according to table 2) was mixed in 1:1 ratio with developer composition 2 (according table 1) and applied onto human hair with 50 % grey hair. After remaining on the hair for 30 minutes, the hair was rinsed with water (T_{water} = 30°C) and dried. The color of the treated hair was colored in fashioned blue-black.

Tint composition 5 (according to table 2) was mixed in 1:1.5 ratio with developer composition 2 (according table 1) and applied onto human hair with 50 % grey hair. After remaining on the hair for 30 minutes, the hair was rinsed with water (T_{water} = 30°C) and dried. The color of the treated hair was colored in fashioned mahogany-red.

Tint compositions 1-5 are according to the invention. Each of these tint compositions performed in various tests as well as a comparative composition comprising phenylene diamines and or resorcinol. With regard to <properties>, the tint compositions .. to .. did perform even better that a comparative composition comprising phenylene diamines and or resorcinol. Tint compositions 2 and 3 do not require any specific labeling on the tube packaging as no resorcinol, no diaminotoluene, no diaminobenzene and no ammonia are present.

### Examples 6-8

**Table 3: Tint compositions (liquid)**

| Tint composition | 6 | 7 | 8 |
|---|---|---|---|
| Oleic Acid | 12.00 | 16.00 | 12.00 |
| Ethoxydiglycol | - | 10.00 | - |
| Undeceth-3 | 10.00 | 14.00 | 14.00 |
| Propylene glycol | 10.00 | 19.00 | 10.00 |
| Cetrimonium chloride | 1.00 | 1.00 | 1.00 |
| Ethyl alcohol | 12.00 | - | 12.00 |
| Laureth-2 | 4.00 | - | 3.00 |
| PEG-15 Cocopolyamine | 2.00 | - | 6.00 |
| Ascorbic acid | 0.30 | 0.30 | 0.30 |
| Sodium sulfite | 0.30 | 0.30 | 0.30 |
| EDTA | 0.20 | 0.20 | 0.20 |
| 2,4,5,6-Tetraaminopyrimidine sulfate | 3.50 | 0.80 | 0.40 |
| 5-Amino-6-chloro-o-cresol | 2.00 | 0.40 | 0.20 |
| 2-Amino-3-hydroxypyridine | 0.45 | 0.90 | 0.21 |
| 4-Amino-m-cresol | 0.30 | 0.95 | 0.20 |
| 2-Amino-5-ethylphenol HCl | - | 0.50 | - |
| 2-Amino-6-chloro-4-nitrophenol | 0.30 | - | 0.025 |
| Ammonia, 28% in water | 6.50 | 8.50 | - |
| Monoethano lamine | - | - | 8.20 |
| | | | |
| Aqua, ad | 100 | 100 | 100 |

Tint composition 6 (according to table 3) was mixed in 1:1 ratio with developer composition 1 (according table 1) and applied onto human hair with 50% grey hair. After remaining on the hair for 30 minutes, the hair was rinsed with water (T_{water} = 30°C) and dried. The color of the treated hair was black.

Tint composition 7 (according to table 3) was mixed in 1:1 ratio with developer composition 2 (according table 1) and applied onto human hair with a grey index of 50%. After remaining on the hair for 30 minutes, the hair was rinsed with water (T_{water} = 30°C) and dried. The color of the treated hair was dark-blond.

Tint composition 8 (according to table 3) was mixed in 1:1 ratio with developer composition 2 (according table 1) and applied onto human hair with a grey index of 50%. After remaining on the hair for 30 minutes, the hair was rinsed with water (T_{water} = 30°C) and dried. The color of the treated hair was light-blond.

Tint compositions 6-8 are according to the invention. Each of these tint compositions performed in various tests as well as a comparative composition comprising phenylene diamines and or resorcinol. With regard to <properties>, the tint compositions .. to .. did perform even better that a comparative composition comprising phenylene diamines and or resorcinol. Tint composition 8 does not require any specific labeling on the tube packaging as no resorcinol, no diaminotoluene, no diaminobenzene and no ammonia are present.

## Claims

1. A cosmetic composition comprising at least these components
i. a first and a second primary intermediate, and
ii. a first and a second coupling agent;
wherein the first primary intermediate is 2,4,5,6-tetraaminopyrimidine or a salt thereof;
wherein the second primary intermediate is selected from the group consisting of p-aminophenol, 4-amino-m-cresol and salts thereof, or a combination of two or more of these;
wherein the first coupling agent is 5-amino-6-chlor-o-cresol; and
wherein the second coupling agent is selected from the group consisting of 2-amino-3-hydroxypyridine, 6-amino-m-cresol, 2-amino-5-ethylphenol and salts thereof, or a combination of two or more of these.

2. The cosmetic composition according to claim 1, wherein the composition comprises at least one of these features:
a) less than 0.1wt.% of a phenylene diamine or salts thereof;
b) less than 0.1wt.% of a resorcinol; or
c) less than 0.2wt.% both, a) and b).

3. The cosmetic composition according to any one of the preceding claims, comprising at least a further coupling agent selected from the group consisting of 3-amino-2,6-dimethylphenol, hydroxyethyl-3,4-methylenedioxyaniline and salts thereof, or a combination of two or more of these group members.

4. The cosmetic composition according to any one of the preceding claims comprising at least one direct dye.

5. The cosmetic composition according to claim 4, wherein the at least one direct dye is selected from the group consisting of 2-amino-6-chloro-4-nitrophenol, 4-nitro-o-phenylenediamine, 2,6-diamino-3-((pyridine-3-yl)azo)pyridine, and salts thereof, or a combination of two or more of these group members.

6. The cosmetic composition of any one of the preceding claims, wherein the composition further comprises at least one organic phosphate ester compound.

7. The cosmetic composition of any one of the preceding claims, wherein the composition further comprises at least one alkaline agent.

8. The cosmetic composition of any of the preceding claims, wherein the composition has at least one of these features:
a. A cumulative amount of all primary intermediates in the range from 0.0001 to 10 wt.%, the wt.% based on the weight of the total composition;
b. A cumulative amount of all coupling agents in the range from 0.0001 to 10 wt.%, the wt.% based on the weight of the total composition;
c. A ratio of the cumulative amounts of all primary intermediates and all coupling agents in the range of from 3:1 to 1:3 [mol/mol];
d. A cumulative amount of all alkaline agents in the range from 0.1 to 17.5 wt.%, the wt.% based on the weight of the total composition;
Or a combination of two or more of the features a. - d..

9. A kit for coloring keratin fibers, comprising in individually packaged form at least two kit components:
I) a cosmetic composition as claimed in any one of claims 1 to 8
II) a developer composition comprising an oxidizing agent.

10. The kit according to claim 9, wherein the oxidizing agent comprises an aqueous solution of hydrogen peroxide.

11. A ready-to-use composition obtainable by mixing the kit components according to any one of claims 9 or 10.

12. The ready-to-use composition according to claim 11, wherein the ratio of component I and component II is in the range of from 2:1 up to 1:3, each number based on parts by weight.

13. A process for coloring keratin fibers, comprising the steps of:
I. providing keratin fibers;
II. contacting the keratin fibers of step I. with the ready-to-use composition according to any one of claims 11 or 12, and allowing the ready-to-use composition to remain on the keratin fibers for a period of time;
III. optionally rinsing the keratin fibers;
IV. optionally drying the keratin fibers.

14. A use of a cosmetic composition according to anyone of claims |1| to |8| for coloring keratin fibers, such as human or animal hair, which are free of resorcinols and phenylene diamines.
